# EUROPÄISCHE PATENTSCHRIFT

(11) **EP 0 568 666 B1**
(45) Veröffentlichungstag und Bekanntmachung des Hinweises auf die Patenterteilung: **24.02.1999**
(21) Anmeldenummer: 92923413.6
(22) Anmeldetag: 20.11.1992
(51) Int. Cl.: A61N 5/06

(54) **VORRICHTUNG ZUR FOTODYNAMISCHEN ENERGIESTIMULIERUNG**
PHOTODYNAMIC STIMULATION DEVICE
DISPOSITIF DE STIMULATION PHOTODYNAMIQUE

(30) Priorität: 20.11.1991 CH 3398/91
(43) Veröffentlichungstag der Anmeldung: 10.11.1993
(73) Patentinhaber: LARSEN, Erik, CH-8201 Schaffhausen (CH)
(72) Erfinder: LARSEN, Erik, CH-8201 Schaffhausen (CH)
(74) Vertreter: Patentanwälte Möll und Bitterich
(86) Internationale Anmeldenummer: CH9200228
(87) Internationale Veröffentlichungsnummer: WO9309847

(56) Entgegenhaltungen:
- EP-A- 0 320 080
- DE-A- 4 012 854
- DE-U- 8 628 810
- DE-U- 8 813 852

## Beschreibung

Die Erfindung betrifft Vorrichtungen zur fotodynamischen Stimulierung von Zellen, insbesondere Zellen menschlichen Gewebes, mittels Lichtstrahlen, gemäß dem Oberbegriff des Anspruchs 1.

Mitochondrien in Zellen von Ein- und Mehrzellern stellen Orte energieliefernder Zellatmung dar. Auch sind sie in der Lage, Proteine zu synthetisieren, da sie über ein vom Zellkern ihrer Zelle unabhängiges, selbständiges genetisches System aus DNA und RNA verfügen. Hauptaufgabe der Mitochondrien ist jedoch die der Zellatmung, d. h. innerhalb der Zellen die Umsetzung von über die Blutbahn und sonstwie zugeführter Nährstoffe und Sauerstoff in Energie und körpereigene Baustoffe, wobei bei der Umsetzung auch Abfallprodukte, wie Wasser, Kohlendioxid, Alkohol und Milchsäure anfallen. Dabei spielt eine bedeutende Rolle die Adenosin-Triphosphorsäure (ATP), die von den Mitochondrien aus Adenosin Diphosphorsäure (ADP) und Orthophosphat synthetisiert wird. Komplizierte chemische Verbindungen spielen dabei als Reaktionskatalysatoren eine entscheidende Rolle.

Aus der EP 0 320 080 A 1 ist eine Vorrichtung zur Biostimulierung von Gewebe bekannt, die Lichtstrahlung mit einer Mehrfachanordnung von im wesentlichen monochromatischen Lichtquellen erzeugt. Als Lichtquellen werden Halbleiterdioden oder Laserdioden vorgeschlagen. Jede Mehrfachanordnung enthält wenigstens drei Lichtquellen, die im wesentlichen monochromatisches Licht im Wellenlängenbereich zwischen 650 nm und 1500 nm abgeben. Die Lichtquellen werden mit Strompulsen von 2,28 Hz bis 400 kHz gepulst. Mit Hilfe eines in die Mehrfachanordnung integrierten Photosensors kann der vom Gewebe reflektierte Anteil der Lichtstrahlung gemessen werden, beispielsweise um die Dauer der Behandlung zu regeln.

Auch aus der DE-U 88 13 852 ist eine Vorrichtung zur fotodynamischen Stimulierung der Energie von lebenden, vorzugsweise nicht pflanzlichen Zellen der oberflächlichen und insbesondere der tieferliegenden Gewebeschichten mittels infraroter Strahlung bekannt. Die Vorrichtung umfaßt ein Energieversorgungs- und Steuergerät und einen Applikator, an dem infrarote Strahlung im 900 nm Bereich abstrahlende IR (Infrarot)-Halbleiterdioden und Reflektoren zur Bündelung der IR-Strahlung vor dem Applikator vorgesehen sind. Das einen Generator enthaltende Steuergerät speist bei dieser bekannten Vorrichtung die Halbleiterdioden mit Strompulsen einer Frequenz, die innerhalb eines Bereiches von 500 - 5000 Hz liegt.

Nachteilig bei dieser Vorrichtung ist, daß die Halbleiterdioden während des Betriebes einer Aufheizung unterliegen, die für die Vorrichtung einen Leistungsabfall nach sich zieht. Die bekannte Vorrichtung gibt mithin während des Betriebs keine konstante Leistung ab. Ein weiterer Nachteil liegt darin, daß infrarote Strahlung in nur einem Wellenlängenbereich von 900 nm verfügbar ist. Leistungsabfall und Strahlung in nur einem Wellenlängenbereich schränken die therapeutische Verwendbarkeit der bekannten Vorrichtung ein.

Hiervon ausgehend, hat sich der Erfinder die Aufgabe gestellt, die bekannten Vorrichtungen zur fotodynamischen Energiestimulierung lebender Zellen dahingehend zu verbessern, daß sie therapeutisch wirksame Lichstrahlung in mindestens zwei Wellenlängenbereich mit konstanter Betriebsleistung abgeben, jedoch mit deutlich reduziertem Aufwand an Lichtquellen.

Diese Aufgabe wird gelöst durch eine Vorrichtung mit Halbleiterdioden gemäß Anspruch 1 und/oder durch eine Vorrichtung mit Laserdioden gemäß Anspruch 2.

Vorteilhafte Weiterbildungen sind Gegenstand der Unteransprüche.

Weitere Vorteile, Merkmale und Einzelheiten der Erfindung ergeben sich aus der folgenden Beschreibung eines bevorzugten Ausführungsbeispiels und der Zeichnungen. Es zeigen
- Fig. 1: eine Darstellung der erfindungsgemäßen Vorrichtung in perspektivischer Darstellung,
- Fig. 2 a, b, c: als Einzelheiten Maschinenapplikatoren der Vorrichtung gemäß Fig. 1,
- Fig. 3: einen Gelenkarm zur bewegbaren Anlenkung der Maschinenapplikatoren,
- Fig. 4: ein Blockschaltbild einer Steuereinheit, die Applikatoren versorgt,
- Fig. 5: einen Handapplikator,
- Fig. 6: einen Applikator gemäß Fig. 5 mit axialer Lichtabstrahlung,
- Fig. 7: einen Applikator gemäß Fig. 5 mit radialer Lichtabstrahlung,
- Fig. 8: einen Applikator mit drehbarem Kopf und
- Fig. 9: als Einzelheit eine Platine für den Appliaktor gemäß Fig. 8.

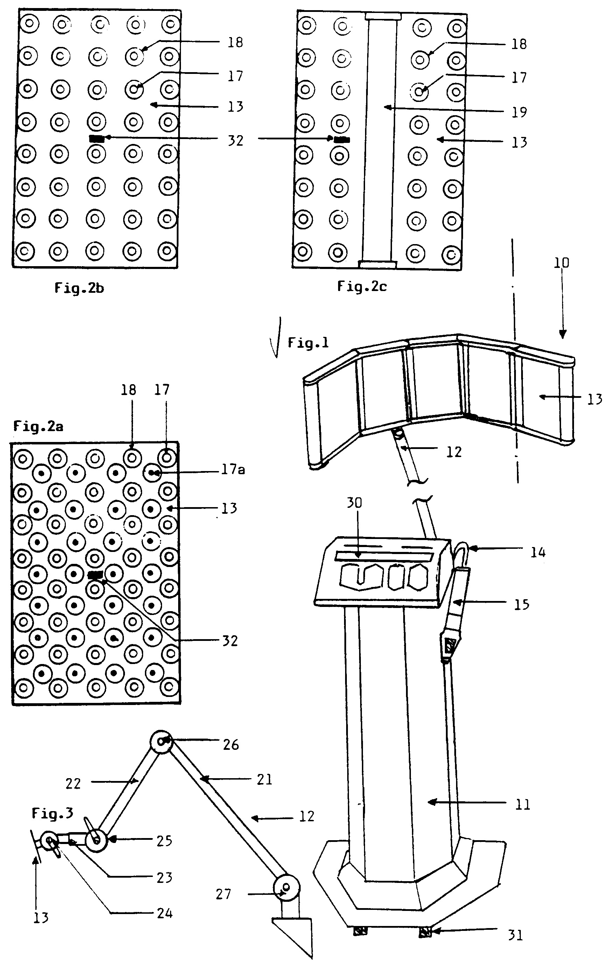

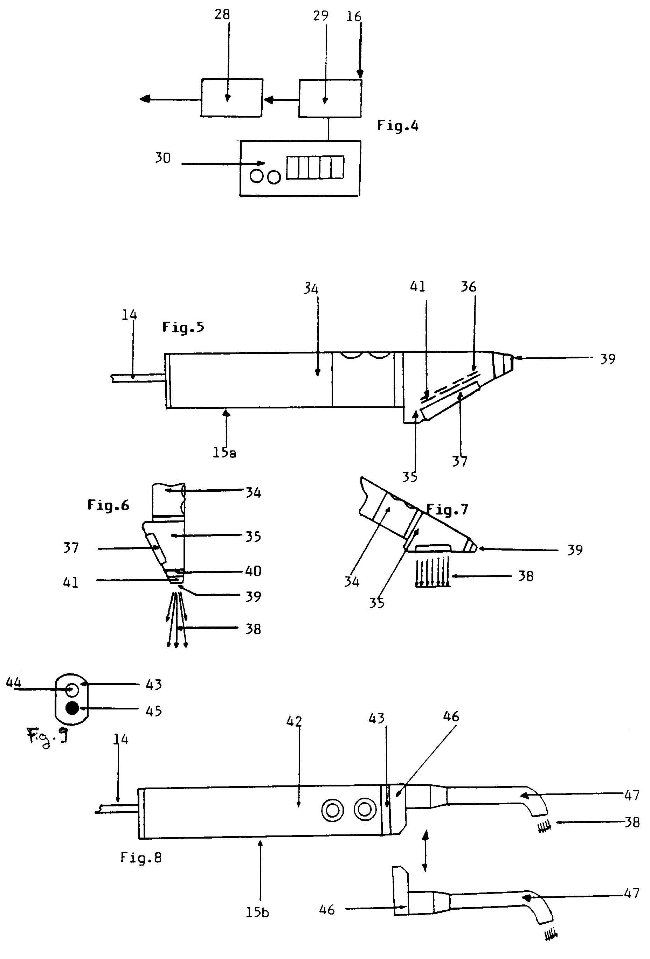

Gemäß Fig. 1 besteht eine Vorrichtung 10 zur fotodynamischen Stimulierung von Zellen aus einer Standsäule 11, an die über einen Gelenkarm 12 Maschinenapplikatoren 13, nachfolgend kurz Applikatoren 13 genannt, angelenkt sind. Die Standsäule 11 ist auch über eine elektrische Leitung 14 mit einem Handapplikator 15 verbunden. Die Standsäule 11, bevorzugt über Rollen 31 frei über eine Auflagefläche bewegbar, enthält das in Fig. 4 dargestellte Steuergerät 16, wobei die Funktionen des Steuergerätes 16 auf einer Steuertafel 30, auch Display 30 genannt, einstell- und überwachbar sind.

Die Fig. 2a, 2b und 2c zeigen flächige Applikatoren 13, die individuell einzeln, mehrfach, nebeneinander oder in anderen Kombinationen zu einem Applikator zusammengestellt sein können. Die Applikatoren 13 sind im Falle der Ausführungsform nach Fig. 2a mit Halbleiterdioden 17 und 17a in versetzter Reihenfolge bestückt, wobei unter versetzter Reihenfolge eine Anordnung verstanden wird derart, daß jeweils eine Diode 17a einer Reihe auf dem Schnittpunkt zweier Diagonalen durch jeweils zwei beidseitig benachbarte Dioden 17 angeordnet ist. Die Dioden 17 und 17a sind mit Reflektoren 18 umgeben, die die Strahlung sammeln und in einer wenige Zentimeter vor dem Applikator 13 liegenden Ebene bündeln.

Gemäß den Fig. 2b und 2c sind die Dioden 17 in regelmäßiger Reihenanordnung, d. h. zueinander aequidistant, angeordnet, wobei nach Fig. 2c ein Applikator 13 zusätzlich eine Lichtquelle 19 aufweist. Die Dioden 17 strahlen Licht mit drei Wellenlängen, nämlich 600, 900 und 1200 nm, also Infrarot-(IR) Strahlung ab, während die Lichtquelle 19 in Form einer Röhre und die Dioden 17a (Fig. 2a) Licht mit einer Wellenlänge von 350 - 500 nm, d. h. Blaulicht, abstrahlen.

Zur Behandlung großflächiger Gewebebereiche sind gemäß Fig. 1 mehrere Applikatoren 13 mit die Längskanten verbindenden Scharnieren 20 beweglich miteinander verbunden, wodurch die Applikatoren an Oberflächengestaltungen von Gewebebereichen, z. B. an Rückenpartien von Menschen, für eine aequidistante Positionierung der Applikatoren 13 einstellbar werden. Der in Fig. 3 gezeigte Gelenkarm 12 verbindet einen oder mehrere Applikator(en) 13 mit der Standsäule 11. Der Gelenkarm 12 weist drei Gelenkträger 21, 22, 23 auf, wobei der Gelenkträger 21 einends mit der Standsäule 11, und der Gelenkträger 23 an einem freien Ende mit einem oder mehreren Applikatoren 13 bewegbar über ein arretierbares Gelenk 24 verbunden ist. Ein weiteres arretierbares Gelenk 25 verbindet den Gelenkträger 23 mit dem Gelenkträger 22, während der Gelenkträger 22 über ein Scharnier 26 mit dem Gelenkträger 21 verbunden ist. Letzterer Gelenkträger 21 ist seinerseits über ein Gelenk 27 mit der Standsäule 11 verbunden. Der Gelenkarm 12 gestattet somit die Positionierung der Applikatoren 13 vor oder über einem Gewebebereich, ohne letzteren zu berühren. Der Gelenkarm 12 trägt weiter die elektrischen Zuleitungen 14 (nicht näher dargestellt) von dem Steuergerät 16 zu dem oder den Applikator(en) 13.

Gemäß Fig. 4 besteht das Steuergerät 16 aus einem Generator 28, einem Zeiteinstellgerät 29 (Timer) und einem Display 30. Vermittels des Generators 8 werden die zur Erzeugung von Licht notwendigen Stromimpulse beigestellt, während über den Timer 29 alle Zeitfunktionen, beispielsweise auch die Behandlungsdauer, einstellbar sind. Das Display 30 zeigt die gewünschten Behandlungsdaten, wie Strompulsfrequenz, Pulslänge und Pulshöhe an. Vermittels des Steuergerätes 16 ist die erfindungsgemäße Vorrichtung bezüglich Länge, Höhe und Frequenz der Strompulse in relativ weiten Bereichen einstellbar, so daß als Lichtquellen Halbleiterdioden, Röhren und Laserdioden verwendbar werden. Zu diesem Zweck ist das Steuergerät für einen Betrieb mit Halbleiterdioden oder Laserdioden auf die eine oder andere Betriebsart, d. h. Halbleiterdiodenbetrieb oder Laserdiodenbetrieb, umschaltbar ausgestaltet.

Für den Halbleiterdiodenbetrieb werden die Dioden 17 und die Dioden 17a für Blaulicht in entsprechender Anpassung mit Strom in Pulsfrequenzen von 200 bis 20 000 Hz mit Strompulslängen zwischen 2 und 20 Mikrosekunden und Spannungspulshöhen zwischen 12 und 25 Volt beaufschlagt. Bei dieser Betriebsweise wird wegen der kurzen Strompulslängen eine Aufheizung wirksam vermieden und dadurch ein leistungskonstanter Betrieb möglich. Gleichzeitig stellen sich bei vorstehender Betriebsart bei jeder Diode 17 gleichzeitig Lichtstrahlungen in drei Wellenlängenbereichen, und zwar in Bereichen von 600, 900 und 1200 nm, ein. Durch gleichzeitige Anregung der Blaulichtdioden 17a stehen dann für therapeutische Zwecke vier Strahlungen mit Wellenlängen von 350 - 500 nm (Blaulicht) sowie 600, 900 und 1200 nm zur Verfügung. Licht im Blaulichtbereich stimuliert Aktivitäten in Zellen und dadurch die Regeneration ermüdeten und kranken Gewebes, insbesondere den Abbau von Fettdepots bei Abmagerungstherapien. Die Hauptstrahlung kommt von den Infrarot-Halbleiterdioden 17. Strahlung im 600 nm-Bereich stimuliert hauptsächlich die Zellatmung oberer Gewebeschichten, während die Strahlung im 900 nm-Bereich die Stimulation von Zellen von der Gewebeoberfläche in bis zu ca. 70 mm tief liegenden Gewebeschichten bewirkt. Die Strahlung im 1200 nm-Bereich dringt noch tiefer als die 900 nm-Strahlung in das Gewebe ein und stimuliert in einem lebenden Körper die Wasserabsorption.

Für den Laserdiodenbetrieb werden Laserdioden 44, 45 als Lichtquellen mit Strompulsen einer Frequenz zwischen 200 und 20 000 Hz, einer Strompulslänge zwischen 2 und 200 Nanosekungen, vorzugsweise zwischen 2 und 20 Nanosekunden, und einer Spannungspulshöhe zwischen 40 und 400 V beaufschlagt. Erzeugt wird ein monochromatisches Laserlicht von ca. 900 nm, das therapeutisch gleich wirksam wie das Licht vergleichbarer Wellenlänge der Halbleiterdioden 17 ist, sofern bei der Laserbetriebsart die Strompulslänge zur fotodynamischen Biostimulation in einem Längenbereich von 100 Nanosekunden gehalten ist. Bei Einstellung kurzer Pulslängen in dem Bereich von 2 bis 20 Nanosekunden und Einstellung einer hohen Betriebsspannung scheidet die Laserdiode 44 Doppelphotonen ab, wodurch eine Blaulichtstrahlung in einem Wellenlängenbereich von ca. 350 bis 500 nm erzeugt wird. Mit Hilfe dieses Zweiphotonenwerkzeugs im nahen Infrarotbereich kann die relativ große Energie des Blaulichtes, die normalerweise bereits auf der Hautoberfläche absorbiert wird, viel tiefer in das Gewebe transportiert werden. Während der Absorption von Doppelphotonen werden im Bereich des Blaulichtes Cytochrome aktiviert und Quantenenergien von 2,8 eV (Elektronenvolt) erreicht. Durch Doppelphotonen wird auch die Aktivität der Chymotrypsin-Enzyme stimuliert.

Die Applikatoren 13 nach den Fig. 2a, 2b und 2c sind mit zwischen den Halbleiterdioden 17, 17a oder Laserdioden 44, 45 angeordneten Sensoren 32 versehen. Die Sensoren 32 haben die Aufgabe zu messen, welche Energiemenge ausgehend von den Applikatoren 13 in J/cm² in das Gewebe eingedrungen ist. Für therapeutische Zwecke ist beispielsweise beabsichtigt, eine vorgegebene Energiemenge pro Flächeneinheit zu bestrahlendem Gewebe einzugeben, die zunächst am Steuergerät 16 eingestellt wird. Sensoren 32 messen nach Aufnahme der energiemäßig voreingestellten Strahlung durch das Gewebe die beispielsweise durch die Hautoberfläche abgestrahlte Energiemenge. Um diese Energiemenge ist die abgestrahlte Energie zu erhöhen, damit die therapeutisch vorgegebene Energiemenge zum Eintrag in das Gewebe gelangt. Die voreingestellte abzüglich abgestrahlte Energiemenge ist diejenige Menge, die in das Gewebe eindringt.

Eine Erhöhung der einzutragenden Energiemenge ist mittels der erfindungsgemäßen Vorrichtung dadurch gegeben, daß entweder die Betriebsspannung (Pulshöhe) oder die Pulsfrequenz oder beide zusammen erhöht und/oder die Behandlungszeit durch Einstellung am Steuergerät 16 verlängert wird.

Während die Applikatoren 13 nach Fig. 2a, 2b und 2c zur Behandlung größerer Gewebeflächen gedacht sind, sind die Handapplikatoren 15a, 15b nach den Fig. 5 und 8 für kleinflächige Gewebebehandlungen vorgesehen.

Der Handapplikator 15a umfaßt einen zylindrischen Schaft 34, an dem ein Kopf 35 angeordnet ist. Im Kopf 35 ist eine Platine 36 mit Lichtquellen, vorzugsweise Halbleiterdioden 17 (nicht dargestellt), vorgesehen. Auch kann auf der Platine 36 eine Blaulicht-Halbleiterdiode 45 angeordnet sein, die in gleicher Weise angeregt wird wie die Dioden der Applikatoren 13, so daß Licht 38 in Wellenlängen von 400, 600, 900 und 1200 nm zur Verfügung steht, das gemäß Fig. 7 aus einem Schlitz 37 aus dem Kopf 35 austritt. Zur Polarisierung der Lichtstrahlen 38 ist vor der Platine 36 ein Polarisationsfilter 41 vorgesehen, dessen Verwendung den Vorteil bietet, daß die Strahlung besser in dem zu behandelnden Gewebe absorbiert wird. Der Kopf 35 weist auch eine vordere Strahlenaustrittsöffnung 39 auf. Im Kopf 35 vor der Austrittsöffnung 39 sind eine Linse 40 zur Fokusierung der Lichtstrahlen 30 und ein Polarisationsfilter 41 vorgesehen, wobei eine Lichtquelle (nicht dargestellt) gemäß Fig. 6 Licht 38 in axialer Richtung durch Linse 40 und Polarisationsfilter 41 abstrahlt. Die Vorrichtung mit dieser Art Lichtabstrahlung eignet sich besonders zur Behandlung kleiner, punktförmiger Gewebeflächen.

Die Fig. 8 und 9 stellen einen Handapplikator 15b dar, der sich besonders für Dentalbehandlungen eignet. Der Applikator 15b weist an dem vorderen Ende seines Schaftes 42 eine Platine 43 auf, die jeweils eine IR-Halbleiterdiode 17 und eine Blaulicht-Halbleiterdiode 17a trägt, wobei die Diode 17 zur Abstrahlung von Licht mit den beschriebenen drei Wellenlängen angeregt wird. Vor der Platine 43 ist ein Kopf 46 mit einem hohlen Ausleger 17 angeordnet, in dem Glasfaser-Lichtkabel (nicht dargestellt) enthalten sind. Der Kopf 46 ist vor der Platine 43 drehbar ausgebildet, so daß der Ausleger 47 vor eine der beiden Dioden 17 oder 17a positonierbar ist. Wird der Ausleger 47 beispielsweise vor die Diode 17 positioniert, so durchläuft Licht der Wellenlängen 600, 900 und 1200 nm das Lichtkabel, wird am vorderen Ende des Auslegers 47 umgelenkt und trifft anschließend auf Gewebe, z. B. Gewebe des Zahnfleisches auf, wodurch schmerzhafte Zahnfleischveränderungen beseitigbar werden. Durch Positionierung des Auslegers 47 vor der Blaulicht-Halbleiterdiode 17a wird Blaulicht durch den Ausleger 47 geleitet, mit dem aus Kunststoff bestehende Zahnfüllungen gehärtet werden können. Es versteht sich, daß auch bei dieser Ausführungsform die Lichtstrahlungen 38 durch Polarisationsfilter geleitet werden können. Auch ist es möglich, beide Handapplikatoren 15a, 15b mit Sensoren 32 wie im Zusammenhang mit dem Applikator 13 beschrieben auzustatten.

## Patentansprüche

1. Vorrichtung zur fotodynamischen Stimulierung von Zellen, insbesondere Zellen menschlichen Gewebes mittels Lichtstrahlen (38), bestehend aus einem Grundgerät (10), umfassend ein Steuergerät (16), einen Generator (28) der Pulse einer Frequenz zwischen 200 und 20.000 Hz und einer Länge zwischen 20 Mikrosekunden und 2 Mikrosekunden abgibt und mindestens einen Applikator (13, 15a, 15b), der wenigstens eine gepulste Lichtquelle (17, 17a, 19) mit Reflektoren (18) zur Bündelung der Lichtstrahlen (38) aufweist, wobei eine Lichtquelle (17) eine Halbleiterdiode (17) ist, die infrarote Strahlung im Wellenbereich von 900 Nanometer abgibt, dadurch gekennzeichnet, daß der Generator (28) zur Speisung der wenigstens einen Lichtquelle (17, 17a) Pulse einer Spannung zwischen 12 und 25 Volt abgibt und daß die Halbleiterdiode (17) infrarote Strahlung in den Wellenbereichen von 600, 900 und 1.200 Nanometer abgibt.

2. Vorrichtung zur fotodynamischen Stimulierung von Zellen, insbesondere Zellen menschlichen Gewebes, mittels Lichtstrahlen (38), bestehend aus einem Grundgerät (10), umfassend ein Steuergerät (16), einen Generator (28) der Pulse einer Frequenz zwischen 200 und 20.000 Hz abgibt und mindestens einen Applikator (13, 15a, 15b), der wenigstens eine gepulste Lichtquelle (44, 45) mit Reflektoren (18) zur Bündelung der Lichtstrahlen (38) aufweist, wobei eine Lichtquelle (44) eine Laserdiode (44) ist, die infrarote Strahlung im Wellenbereich von 900 Nanometer abgibt, dadurch gekennzeichnet, daß der Generator (28) zur Speisung der wenigstens einen Lichtquelle (17, 17a) Pulse einer Spannung zwischen 40 und 400 Volt und einer Länge zwischen 20 Nanosekunden und 2 Nanosekunden abgibt zur Anregung einer Doppelphotonenabscheidung.

3. Vorrichtung nach Anspruch 1 oder 2, dadurch gekennzeichnet, daß zu den Lichtquellen Halbleiterdioden (17a) und/oder Laserdioden (45) gehören, die Blaulichtstrahlung im Wellenbereich von 350 bis 500 Nanometer abgeben.

4. Vorrichtung nach einem der Ansprüche 1 oder 2, dadurch gekennzeichnet, daß zu den Lichtquellen eine Röhre (19) gehört, die Blaulichtstrahlung im Wellenbereich von 350 bis 500 Nanometer abgibt.

5. Vorrichtung nach einem der Ansprüche 1 bis 4, dadurch gekennzeichnet, daß an den Applikatoren (13, 15a, 15b) Sensoren (32) angebracht sind, die die reflektierte Lichtstrahlung messen.

6. Vorrichtung nach einem der Ansprüche 1 bis 7, dadurch gekennzeichnet, daß wenigstens ein Applikator (13) aus mehreren flächigen Einzelapplikatoren besteht, die über Scharniere (20) zueinander im Winkel verstellbar sind.

7. Vorrichtung nach Anspruch 6, dadurch gekennzeichnet, daß der Applikator (13) über einen Gelenkarm (12) an einer Standsäule (11) angelenkt ist.

8. Vorrichtung nach einem der Ansprüche 1 bis 5, dadurch gekennzeichnet, daß wenigstens ein Handapplikator (15a, 15b) mit einem Schaft (34, 42) und einem Kopf (35, 46) vorgesehen ist und daß im Kopf (35, 46) eine Platine (36, 43) mit den Halbleiter- und/oder Laserdioden (17, 17a, 44, 45) untergebracht ist.

9. Vorrichtung nach Anspruch 8, dadurch gekennzeichnet, daß im Kopf (35) ein Schlitz (37) vorgesehen ist, durch den die Lichtstrahlung (38) austreten kann.

10. Vorrichtung nach Anspruch 8 oder 9, dadurch gekennzeichnet, daß im Kopf (35) eine axial gerichtete Strahlenaustrittsöffnung (39) mit einer Linse (40) vorgesehen ist.

11. Vorrichtung nach Anspruch 8, 9 oder 10, dadurch gekennzeichnet, daß den Dioden (17, 17a, 44, 45) ein Polarisationsfilter (41) vorgesetzt ist.

12. Vorrichtung nach einem der Ansprüche 8 bis 11, dadurch gekennzeichnet, daß an dem Kopf (46) ein Ausleger (47) angeformt ist und daß der Kopf (46) vor der Platine (43) derart drehbar ist, daß entweder Infrarotlicht oder Blaulicht über den Ausleger (47) zu einem Behandlungsort leitbar ist.

13. Vorrichtung nach Anspruch 12, dadurch gekennzeichnet, daß der Ausleger (47) ein lichtleitendes Glasfaserkabel enthält.

## Claims

1. An apparatus for the photodynamic stimulation of cells, in particular cells of human tissue, by means of light beams (38), consisting of a basic device (10) comprising a control device (16), a generator (28) which emits pulses of a frequency of between 200 and 20,000 Hz and a length of between 20 microseconds and 2 microseconds, and at least one applicator (13,15a,15b) which has at least one pulsed light source (17,17a,19) with reflectors (18) for focusing the light beams (38), wherein one light source (17) is a semiconductor diode (17) which emits infrared radiation in a waveband of 900 nanometers, characterised in that for feeding the at least one light source (17,17a) the generator (28) emits pulses of a voltage of between 12 and 25 volts, and in that the semiconductor diode (17) emits infrared radiation in the wavebands of 600, 900 and 1,200 nanometers.

2. An apparatus for the photodynamic stimulation of cells, in particular cells of human tissue, by means of light beams (38), consisting of a basic device (10) comprising a control device (16), a generator (28) which emits pulses of a frequency of between 200 and 20,000 Hz, and at least one applicator (13,15a,15b) which has at least one pulsed light source (44,45) with reflectors (18) for focusing the light beams (38), wherein one light source (44) is a laser diode (44) which emits infrared radiation in a waveband of 900 nanometers, characterised in that for feeding the at least one light source (17,17a) the generator (28) emits pulses of a voltage of between 40 and 400 volts, and a length of between 20 nanoseconds and 2 nanoseconds so as to stimulate double photon detachment.

3. An apparatus according to Claim 1 or 2, characterised in that the light sources are associated with semiconductor diodes (17a) and/or laser diodes (45) which emit blue light radiation at a waveband of 350 to 500 nanometers.

4. An apparatus according to Claim 1 or 2, characterised in that the light sources are associated with a tube (19) which emits blue light radiation in the waveband of 350 to 500 nanometers.

5. An apparatus according to any one of Claims 1 to 4, characterised in that sensors (32) are applied to the applicators (13,15a,15b), which sensors measure the reflector light radiation.

6. An apparatus according to any one of Claims 1 to 5, characterised in that at least one applicator (13) comprises a plurality of planar individual applicators which can be adjusted at angle to one another via hinges (20).

7. An apparatus according to Claim 6, characterised in that the applicator (13) is articulated to a vertical column via a pivot arm (12).

8. An apparatus according to any one of Claims 1 to 5, characterised in that at least one manual applicator (15a,15b) is provided with a shank (34,42) and a head (35,46), and that a board (36,43) with the semiconductor diodes and/or laser diodes (17,17a,44,45) is accommodated in the head (35,46).

9. An apparatus according to Claim 8, characterised in that a slot (37) is provided in the head (35), through which the light radiation (38) can emerge.

10. An apparatus according to Claim 8 or 9, characterised in that an axially directed radiation outlet opening (39) with a lens (40) is provided in the head.

11. An apparatus according to Claim 8, 9 or 10, characterized in that a polarising filter (41) is disposed in front of the diodes (17,17a,44,45).

12. An apparatus according to any one of Claims 8 to 11, characterized in that an arm (47) is formed on the head (46), and in that the head (46) can be rotated in front of the board (43) in such a way that either infrared light or blue light can be conducted via the arm (47) to the treatment site.

13. An apparatus according to Claim 12, characterized in that the arm (47) contains a light-conducting glass-fibre cable.

## Revendications

1. Dispositif de stimulation photodynamique de cellules, en particulier les cellules du tissu humain, à l'aide de rayons lumineux (38), comprenant un appareil de base (10) formé par un appareil de commande (16), un générateur (28) qui émet des impulsions avec une fréquence comprise entre 200 et 20 000 Hz et une longueur comprise entre 20 microsecondes et 2 microsecondes, et au moins un applicateur (13, 15a, 15b) qui comporte au moins une source de lumière à régime impulsionnel (17, 17a, 19) avec des réflecteurs (18) pour focaliser les rayons lumineux (38), une source lumineuse (17) étant constituée par une diode à semi-conducteur (17), qui émet un rayon infrarouge dans la plage de longueur d'ondes de 900 nanomètres, caractérisé en ce que le générateur (28) émet des impulsions comprises entre 12 et 25 volts pour alimenter au moins une source lumineuse (17, 17a) et en ce que la diode à semi-conducteur (17) émet un rayon infrarouge dans les plages de longueur d'ondes de 600, 900 et 1 200 nanomètres.

2. Dispositif de stimulation photodynamique de cellules, en particulier les cellules du tissu humain, à l'aide de rayons lumineux (38), comprenant un appareil de base (10) formé par un appareil de commande (16), un générateur (28) qui émet des impulsions avec une fréquence comprise entre 200 et 20 000 Hz et au moins un applicateur (13, 15a, 15b) qui comporte au moins une source de lumière à régime impulsionnel (44, 45) avec des réflecteurs (18) pour focaliser les rayons lumineux (38), une source lumineuse (44) étant constituée par une diode à laser (44), qui émet un rayon infrarouge dans la plage de longueur d'ondes de 900 nanomètres, caractérisé en ce que le générateur (28) émet des impulsions avec une tension comprise entre 40 et 400 volts pour alimenter au moins une source lumineuse (17, 17a) et avec une longueur comprise entre 20 nanosecondes et 2 nanosecondes pour déclencher une double photoséparation.

3. Dispositif selon la revendication 1 ou 2, caractérisé en ce que les sources lumineuses contiennent des diodes à semi-conducteur (17a) et/ou des diodes laser (45), qui émettent un rayon de luminescence bleue dans la plage de longueur d'ondes comprise entre 350 et 500 nanomètres.

4. Dispositif selon la revendication 1 ou 2, caractérisé en ce que les sources lumineuses contiennent un tube à vide (19) qui émet un rayon de luminescence bleue dans la plage de longueur d'ondes comprise entre 350 et 500 nanomètres.

5. Dispositif selon l'une quelconque des revendications 1 à 4, caractérisé en ce que des capteurs (32) sont montés contre les applicateurs (13, 15a, 15b), lesquels capteurs mesurent le rayon lumineux réfléchi.

6. Dispositif selon l'une quelconque des revendications 1 à 5, caractérisé en ce qu'au moins un applicateur (13) est constitué par plusieurs applicateurs séparés plats, qui peuvent être décalés en formant un angle les uns avec les autres à l'aide de charnières (20).

7. Dispositif selon la revendication 6, caractérisé en ce que l'applicateur (13) est articulé contre une colonne fixe (11) par l'intermédiaire d'un bras articulé (12).

8. Dispositif selon l'une quelconque des revendications 1 à 5, caractérisé en ce qu'est prévu au moins un applicateur à manoeuvre manuelle (15a, 15b) comprenant une tige (34, 42) et une tête (35, 46) et en ce qu'une platine (36, 43), munie des diodes à semi-conducteur et/ou des diodes laser (17, 17a, 44, 45), est montée dans la tête (35, 46).

9. Dispositif selon la revendication 8, caractérisé en ce qu'est prévue, dans la tête (35), une fente (37) par laquelle peut passer le rayon lumineux (38).

10. Dispositif selon la revendication 8 ou 9, caractérisé en ce qu'est prévue, dans la tête (35), un orifice de passage (39) pour les rayons, orienté dans le sens axial et muni d'une lentille (40).

11. Dispositif selon la revendication 8, 9 ou 10, caractérisé en ce qu'un filtre de polarisation (41) est monté en amont des diodes (17, 17a, 44, 45).

12. Dispositif selon l'une quelconque des revendications 8 à 11, caractérisé en ce qu'un bras (47) est formé contre la tête (46) et en ce que la tête (46) peut pivoter devant la platine (43) de telle sorte que soit la lumière infrarouge, soit la luminescence bleue est guidée par l'intermédiaire du bras (47) vers une zone de traitement.

13. Dispositif selon la revendication 12, caractérisé en ce que le bras (47) contient un câble à fibre de verre guidant la lumière.
